(19) Europäisches Patentamt
European Patent Office
Office européen des brevets

(11) Numéro de publication: 0 384 812
A1

# (12) DEMANDE DE BREVET EUROPEEN

(21) Numéro de dépôt: 90400429.8

(51) Int. Cl.[5] C07D 235/30

(22) Date de dépôt: 16.02.90

(30) Priorité: 20.02.89 FR 8902168

(43) Date de publication de la demande:
29.08.90 Bulletin 90/35

(84) Etats contractants désignés:
AT BE CH DE DK ES FR GB GR IT LI LU NL SE

(71) Demandeur: RHONE-POULENC SANTE
20, avenue Raymond Aron
F-92160 Antony(FR)

(72) Inventeur: Audiau, François
9 rue Guérin
F-94220 Charenton Le Pont(FR)
Inventeur: Renault, Christian
61 Rue des Mallets
F-95150 Taverny(FR)

(74) Mandataire: Savina, Jacques et al
RHONE-POULENC SANTE, Service Brevets,
20 Avenue Raymond Aron
F-92165 Antony Cédex(FR)

## (54) Trifluorométhoxy-5 benzimidazolamine-2, procédé pour sa préparation et médicaments la contenant.

(57) La trifluorométhoxy-5 benzimidazolamine-2 et ses sels avec un acide minéral ou organique, sa préparation et les médicaments la contenant.

EP 0 384 812 A1

Xerox Copy Centre

# TRIFLUOROMETHOXY-5 BENZIMIDAZOLAMINE-2, PROCEDE POUR SA PREPARATION ET MEDICAMENTS LA CONTENANT

La présente invention concerne la trifluorométhoxy-5 benzimidazolamine-2, sa préparation et les médicaments la contenant.

La trifluorométhoxy-5 benzimidazolamine-2 est représentée par la formule :

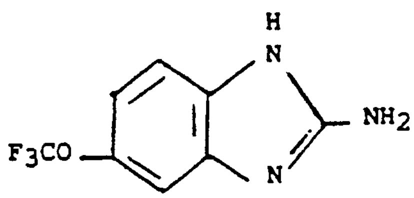

(I)

Les sels du composé de formule (I) avec des acides minéraux ou organiques font également partie de l'invention.

Le composé de formule (I) peut être préparé par action du bromure de cyanogène sur la trifluorométhoxy-4 benzènediamine-1,2.

Ce procédé consiste en une adaptation de la méthode décrite par R.C. EDERFIELD, Heterocyclic Compounds, 1957, 5, 285 pour la préparation de la benzimidazolamine-2.

La réaction du bromure de cyanogène sur la trifluorométhoxy-4 benzènediamine-1,2 peut être effectuée vers 0 °C dans un milieu inerte hydroorganique. Comme solvant organique, on peut utiliser le tétrachloroéthane.

Le bromure de cyanogène peut être généré in situ par action de brome sur le cyanure de sodium selon la méthode décrite dans Organic Synthesis, 1983, 61,35.

Le mélange réactionnel obtenu est traité suivant des méthodes classiques physiques (extraction, cristallisation, chromatographie) ou chimiques (formation de sel et régénération de la base) afin d'isoler le composé de formule (I) à l'état pur.

Le composé de formule (I) sous forme de base libre peut être éventuellement transformé en sel d'addition avec un acide minéral ou organique par action d'un tel acide au sein d'un solvant organique inerte tel qu'un alcool, une cétone, un éther ou un solvant chloré.

Le composé de formule (I) et ses sels présentent des propriétés pharmacologiques intéressantes. Ce composé est un antagoniste à l'égard de l'acide aspartique et est actif vis-à-vis des réponses électrophysiologiques provoquées chez le rat par applicastion iontophorétique d'acides aminés neuroexcitateurs, notamment l'acide kainique. Ce composé est donc utile pour le traitement de troubles neurologiques et psychiatriques, en particulier les états psychotiques (schizophrénie), l'épilepsie, les maladies neurodégénératives telles que la maladie d'Alzheimer ou la maladie du Huntington et l'ischémie cérébrale.

Les propriétés antagonistes à l'égard de l'acide aspartique ont été déterminées en mesurant l'inhibition de l'augmentation du taux de guanosine monophosphate (GMP) cyclique induit par l'acide aspartique selon la méthode de J. GARTHWAITE, Neuroscience, 1982, 7, 2491-2497.

Des rats jeunes et mâles (8-12 jours) sont décapités et le cervelet est rapidement prélevé et placé dans un tampon à 0 °C (122 NaCl, 3 mM KCl, 1,2 mM $MgSO_4$, 1,3 mM $CaCl_2$, 0,4 mM $kH_2$ $PO_4$, 25 mM NaH $CO_3$ et 10 mM glucose, contenant 0,5 mM de théophyline pour éviter la dégradation enzymatique du GMP cyclique). Des coupes du cervelet (0,6-0,8 mm) sont incubées pendant 40 mn. à 37 °C sous atmosphère de 95 % $0_2$, 5 % $CO_2$ dans 20 $cm^3$ du même tampon, puis lavées et placées à nouveau dans 15 $cm^3$ de tampon dans les même conditions pendant 60 mn.

Les coupes sont à nouveau lavées et transférées dans 15 $cm^3$ de tampon contenant les produits à étudier. Après 18 mn. d'incubation les tissus sont broyés dans 2 $cm^3$ d'éthanol et le taux de GMP cyclique est déterminé par dosage radioimmunologique selon une méthode décrite par A. L.STEINER, C.W. PARKER et D.M. KIPNIS, J. Biol. Chem. 1972, 247, 1106.

L'acide aspartique est utilisé à la concentration de 100 µM et le produit à étudier est ajouté 3 mn. avant l'acide aspartique à une concentration de 25,30 ou 100 µM. Les résultats utilisés sont la moyenne de 5 à 10 expériences pour chaque concentration et on détermine une $IC_{50}$ qui est la concentration inhibant de 50 % l'augmentation du taux de cyclique induit par l'acide aspartique.

Le composé de formule (2) présente dans ce test une $IC_{50}$ de 30 µM.

L'activité vis-à-vis des réponses électrophysiologiques provoquées chez le rat par application iontophorétique d'acides aminés neuroexcitateurs tels que les acides N-méthyl D-aspartique (NMDA), kainique et quisqualique (QI) est déterminée selon la méthode de M. A. MARTIN et al., European Journal of Pharmacology, 1977, VOL.42, 291-298.

A la dose de 1 mg kg, le composé de formule (I) provoque, 30 minutes après son administration, une diminution de 90 % des réponses électrophysiologiques au kainate et de 20 % des réponses au NMDA et au QI.

Le composé selon l'invention et ses sels présentent une faible toxicité. La $DL_{50}$ par voie orale chez la souris du composé de l'exemple 1 est de 620 mg/kg. Cette $DL_{50}$ a été calculée, après 3 jours d'observation par la méthode cumulative de J.J. REED et H. MUENCH, Amer. J. Hyg., 1938, 27, 493.

Pour l'emploi médicinal, il peut être fait usage du produit de formule (I) tel quel ou à l'état de sel avec un acide pharmaceutiquement acceptable.

Comme sels pharmaceutiquement acceptables, on peut citer les sels d'addition avec un acide minéral tels que le chlorohydrate, le sulfate, le nitrate ou le phosphate ou avec un acide organique tels que l'acétate, le propionate, le succinate, le benzoate, le fumarate, le théophillineacétate, le salicylate, le méthylène bis (z-hydroxynaphtoate) ou des dérivés de substitution de ces produits.

## EXEMPLE 1

A un mélange refroidi à 0 °C de 5,8 g de brome et de 5,6 $cm^3$ d'eau, on ajoute goutte à goutte 1,79 g de cyanure de sodium en solution dans 5,6 $cm^3$ d'eau. Après 1 heure de contact à 0 °C, on ajoute goutte à goutte une solution de 7 g de trifluorométhoxy-4 benzènediamine-1,2 dans 7 $cm^3$ de tétrachloroéthane. Après 2 heures à 0 °C, on verse le mélange réactionnel dans 500 $cm^3$ d'eau. On ajoute 500 $cm^3$ de dichlorométhane et on agite 20 minutes. On décante la phase aqueuse, la lave avec 2 fois 150 $cm^3$ de dichlorométhane et l'alcalinise à pH 8 avec du carbonate de sodium. On extrait la base avec 3 fois 300 $cm^3$ d'acétate d'éthyle, sèche la phase organique sur sulfate de magnésium et évapore sous pression réduite. On obtient 9,5 g de base brute que l'on purifie par HPLC préparative en utilisant un mélange dichlorométhane-éthanol (95-5 en volumes) comme éluant. On récupère une huile que l'on transforme en chlorhydrate au sein d'éther éthylique. On obtient 4,8 g de chlorhydrate de trifluorométhoxy-5 benzimidazolamine-2 fondant à 180 °C.

La trifluorométhoxy- 4 benzènediamine- 1,2 est préparée selon Zhur. Obshchei. Khim., 1961, 31, 915.

Les médicaments selon l'invention sont constitués par le composé de formule (I) ou un sel de ce composé, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennnent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, le composé selon l'invention est particulièrement utile pour le traitement des troubles neurologiques et psychiatriques, en particulier les états psychotiques (schizophrénie), l'épilepsie, les maladies neurodégénératives telles que la maladie d'Alzheimer ou la maladie de Huntington et l'ischémie cérébrale.

Les doses dépendent de l'effet recherché, de la durée du traitement et de la voie d'administration utilisée : elles sont généralement comprises entre 50 et 500 mg par jour par voie orale pour un adulte avec des doses unitaires allant de 10 à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

Exemple A

On prépare, selon la technique habituelle, des gélules dosées à 25 mg de produit actif ayant la composition suivante :

| | |
|---|---|
| - Trifluorométhoxy-5 benzimidazolamine-2 ..... | 25 mg |
| - Cellulose microcristalline ..... | 75 mg |
| - Mannitol ..... | 41 mg |
| - Silice colloïdale ..... | 4 mg |
| - Carboxyméthylamidon sodique ..... | 25 mg |
| - Talc ..... | 18 mg |
| - Stéarate de magnésium ..... | 2 mg |
| - Polyvidone excipient ..... | 10 mg |

Exemple B

On prépare selon la technique habituelle, des comprimés dosés à 50 mg de produit actif ayant la composition suivante

| | |
|---|---|
| - Trifluorométhoxy-5 benzimidazolamine-2 ..... | 50 mg |
| - Cellulose microcristalline ..... | 75 mg |
| - Mannitol ..... | 41 mg |
| - Polyvidone excipient ..... | 10 mg |
| - Carboxyméthylamidon ..... | 25 mg |
| - Silice colloïdale ..... | 4 mg |
| - Talc ..... | 18 mg |
| - Stéarate de magnésium ..... | 2 mg |
| - Mélange d'hydroxyméthylcellulose, glycérine, oxyde de titane (72-3,5-24,5) .....q.s.p. | 1 comprimé pelliculé terminé à 245 mg |

Exemple C

On prépare une solution injectable contenant 10 mg de produit actif ayant la compositon suivante :

| | |
|---|---|
| - Trifluorométhoxy-5 benzimidazolamine-2 ..... | 10 mg |
| - Acide benzoïque ..... | 80 mg |
| - Alcool benzylique ..... | 0,06 $cm^3$ |
| - Benzoate de sodium ..... | 80 mg |
| - Ethanol à 95 % ..... | 0,4 $cm^3$ |
| - Hydroxyde de sodium ..... | 24 mg |
| - Propylèneglycol ..... | 1,6 $cm^3$ |
| - Eau .....q.s.p. | 4 $cm^3$ |

## Revendications

1 - La trifluorométhoxy-5 benzimidazolamine-2 ou ses sels avec un acide minéral ou organique.

2 - Procédé de préparation du composé selon la revendication 1 caractérisé en ce que l'on fait réagir le bromure de cyanogène sur la trifluorométhoxy-4 benzènediamine-1,2, isole le produit et le transforme éventuellement en sel avec un acide minéral ou organique.

3 - Médicament contenant au moins une substance active et éventuellement un ou plusieurs diluants ou adjuvants compatibles et pharmaceutiquement acceptables caractérisé en ce que la substance active est la trifluorométhoxy-5 benzimidazolamine-2 ou un de ses sels avec un acide minéral ou organique.

Revendications pour les Etats contractants suivants: GR, ES

Procédé de préparation de la trifluorométhoxy-5 benzimidazolamine-2 ou ses sels avec un acide minéral ou organique caractérisé en ce que l'on fait réagir le bromure de cyanogène sur la trifluométhoxy-4 benzénediamine-1,2, isole le produit et le transforme éventuellement en sel avec un acide minéral ou organique.

Office européen des brevets

# RAPPORT DE RECHERCHE EUROPEENNE

Numero de la demande

EP 90 40 0429

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 85, no. 9, 30 août 1976, page 566, résumé no. 63069e, Columbus, Ohio, US; K. SAWATARI et al. (Yoshitomi Pharmaceutical Industries, Ltd): "2-Aminobenzimidazoles" * Abrégé * --- | | C 07 D 235/30 |
| A | CH-A- 667 649 (LONZA AG) --- | | |
| A | EP-A-0 091 796 (ELI LILLY AND CO.) --- | | |
| A | US-A-3 455 948 (ROBERT JOHN STEDMAN (SMITH KLINE & FRENCH LABORATORIES)) ----- | | |

| DOMAINES TECHNIQUES RECHERCHES (Int. Cl.5) |
|---|
| C 07 D 235/00 |

Le présent rapport a été établi pour toutes les revendications

| Lieu de la recherche | Date d'achèvement de la recherche | Examinateur |
|---|---|---|
| LA HAYE | 02-04-1990 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant